# EUROPEAN PATENT SPECIFICATION

(11) **EP 2 995 326 B1**
(45) Date of publication and mention of the grant of the patent: **24.07.2019**
(21) Application number: 14795291.5
(22) Date of filing: 02.05.2014
(51) Int. Cl.: A61L 27/36, A61K 47/00, A61K 35/00, A61L 15/00

(54) **HYBRID GEL CONTAINING PARTICULATE DECELLULARIZED TISSUE**
HYBRIDGEL MIT PARTIKELFÖRMIGEM DEZELLULARISIERTEM GEWEBE
GEL HYBRIDE CONTENANT UN TISSU DÉCELLULARISÉ PARTICULAIRE

(30) Priority: 07.05.2013 JP 2013097399
(43) Date of publication of application: 16.03.2016
(73) Proprietor: KM Biologics Co., Ltd., Kumamoto 860-8568 (JP); National University Corporation Tokyo Medical and Dental University, Bunkyo-ku Tokyo 113-8510 (JP); Sapporo Medical University, Sapporo-shi, Hokkaido 060-8556 (JP); ADEKA CORPORATION, Arakawa-ku Tokyo 116-8554 (JP)
(72) Inventor: MATSUDA, Junichi, Kikuchi-shi Kumamoto 869-1298 (JP); MIYABASHIRA, Sumika, Kikuchi-shi Kumamoto 869-1298 (JP); HARANO, Satomi, Kikuchi-shi Kumamoto 869-1298 (JP); KISHIDA, Akio, Tokyo 113-8510 (JP); KIMURA, Tsuyoshi, Tokyo 113-8510 (JP); NEGISHI, Jun, Tokyo 113-8510 (JP); HIGAMI, Tetsuya, Sapporo-shi Hokkaido 060-8556 (JP); FUNAMOTO, Seiichi, Sapporo-shi Hokkaido 060-8556 (JP); HIWATARI, Ken-ichiro, Tokyo 116-8554 (JP); TASAKI, Akiko, Tokyo 116-8554 (JP)
(74) Representative: Vossius & Partner Patentanwälte Rechtsanwälte mbB
(86) International application number: PCT/JP2014/062544
(87) International publication number: WO 2014/181886

(56) References cited:
- EP-A1- 2 944 330
- EP-A1- 2 995 325
- EP-A2- 2 345 435
- WO-A1-03/032735
- WO-A2-2012/027514
- JP-A- 2005 506 121
- SINGELYN J M ET AL: "Naturally derived myocardial matrix as an injectable scaffold for cardiac tissue engineering", BIOMATERIALS, ELSEVIER SCIENCE PUBLISHERS BV., BARKING, GB, vol. 30, no. 29, 1 October 2009 (2009-10-01), pages 5409-5416, XP026469964, ISSN: 0142-9612, DOI: 10.1016/J.BIOMATERIALS.2009.06.045 [retrieved on 2009-07-15]
- WOLF MATTHEW T ET AL: "A hydrogel derived from decellularized dermal extracellular matrix", BIOMATERIALS, vol. 33, no. 29, 11 July 2012 (2012-07-11) , pages 7028-7038, XP028932396, ISSN: 0142-9612, DOI: 10.1016/J.BIOMATERIALS.2012.06.051
- JUN NEGISHI ET AL: "Effect of treatment temperature on collagen structures of the decellularized carotid artery using high hydrostatic pressure", JOURNAL OF ARTIFICIAL ORGANS ; THE OFFICIAL JOURNAL OF THE JAPANESE SOCIETY FOR ARTIFICIAL ORGANS, SPRINGER-VERLAG, TO, vol. 14, no. 3, 11 May 2011 (2011-05-11), pages 223-231, XP019948540, ISSN: 1619-0904, DOI: 10.1007/S10047-011-0570-Z
- LIN PAN B ET AL.: 'A study on repair of porcine articular cartilage defects with tissue- engineered cartilage constructed in vivo by composite scaffold' ANNALS OF PLASTIC SURGERY vol. 65, no. 4, October 2010, pages 430 - 6, XP008182345
- BANNASCH, H ET AL.: 'Wound healing in a pig animal model after transplantation of autologous keratinocytes suspended in fibrin glue and decellularised dermis' CHIRURGISCHES FORUM FUER EXPERIMENTELLE UND KLINISCHE FORSCHUNG 2002, pages 485 - 487, XP008182364

## Description

### TECHNICAL FIELD

The present invention is defined by the claims and is generally in the technical field of regenerative therapy. Specifically, the present invention relates to a hybrid gel comprising a particulate decellularized tissue obtained by pulverizing animal-derived biological tissues that are decellularized (decellularized biological tissues), fibrinogen and thrombin, wherein thrombin is at a concentration of 0.8 U/mL to 125 U/mL and fibrinogen is at a concentration of 30 mg/mL to 40 mg/mL, and a method for utilizing the same.

### BACKGROUND ART

When a graft is transplanted from biological tissues of others, rejection by the tissue of the subject who received the graft becomes a problem. For solving such a problem, development of an artificial tissue is expected. As material, a variety of macromolecules have been attempted. However, since compatibility between such material and biological tissues is low, a graft may be dropped out from the joining site or infectious diseases may occur.

Thus, in order to improve compatibility with biological tissues, the techniques have been developed to use decellularized biological tissues, which are supporting tissues that remain after removal of cells from biological tissues, as a graft, and to use a resulting material obtained by pulverizing such decellularized biological tissues (a particulate decellularized tissue).

Among these, CryoLife has developed a human-derived decellularized heart valve that can firstly commercially be used and AutoTissue has developed a pig-derived decellularized heart valve for the purpose of treating human heart diseases.

Concerning a decellularized tissue and a particulate decellularized tissue, there are the following reports. Non-patent reference 1 (Sasaki S., et al., Mol. Vis., 2009, 15, 2022-2028) and Non-patent reference 2 (Hashimoto Y., et al., Biomaterials, 2010, 31, 3941-3948) reported that the pig cornea that are decellularized by a high hydrostatic pressure treatment was functional when transplanted to the eye of rabbits. Patent reference 1 (Japanese Patent Publication No. 2013-42677) discloses a process liquid suitable for decellularization treatment of the cornea. Non-patent reference 3 (Funamoto S., et al., Biomaterials, 2010, 31, 3590-3595) reported that the pig-derived aorta was decellularized by a high hydrostatic pressure treatment and the resulting decellularized tissue was estimated for its mechanical property and in vivo ability to reveal that the mechanical property was not influenced by a high hydrostatic pressure treatment, that an inflammatory reaction was restrained, that the tissue could resist against the blood pressure of the arteries and that blood clot was not formed in the luminal surface. Non-patent reference 3 also reported that cellular infiltration into the vascular wall occurred four weeks after transplant of said blood vessel. Non-patent reference 4 (Negishi J., et al., J. Artif. Organs, 2011, 14, 223-231) reported that, when the rat-derived carotid artery was decellularized under conditions where degeneration of collagen is restricted and the decellularized artery was transplanted to rat, arterial occlusion was restricted. WO 2012/027514 relates to a composition comprising a decellularized matrix obtained from cardiac tissue and having a molecular weight of less than 300 kDa in combination with a fibrin glue for cardiac therapy. WO 2003/032735 describes an animal tissue micronized acellular matrix which was implanted along with a fibrin glue for the correction of osteochondral defects. Lin et al. (2010) ANNALS OF PLASTIC SURGERY, 65(4):430-436 refers to a method aiming at repairing tissue defects comprising the injection of a microparticular acellular tissue matrix and fibrin glue into articluar cartilage defective regions.

Non-patent reference 5 (Singelyn J.M., et al., Biomaterials, 2009, 30, 5409-5416) reported that a heart muscle matrix (obtained by decellularizing the pig heart muscle with a surfactant, followed by lyophilization, pulverization and pepsin digestion) self-associated to form a porous, fibrous hydrogel and that endothelial cells and smooth muscle cells infiltrated to said hydrogel to increase the formation of small vessels. Non-patent reference 6 (Seif-Naraghi S., et al., J. Vis. Exp., 2010, 46, e2109) examined a host response after said hydrogel was applied to the free wall of the left ventricle. Non-patent reference 7 (Singelyn J.M., et al., J. Am. Coll. Cardiol., 2012, 59, 751-763) reported that, when said hydrogel was applied to a myocardial infarction model rat, intrinsic myocardial cells increased and a cardiac function was maintained without causing arrhythmia. Non-patent reference 8 (Sonya B., et al., Sci. Transl. Med., 2013, 5, 173ra25) reported that, when said hydrogel was applied to a pig myocardial infarction model, a cardiac function, a volume of the ventricles and a general movement of the cardiac wall improved and that said hydrogel was biocompatible to rat. Non-patent reference 9 (Wolf M.T., et al., Biomaterials, 2012, 33, 7028-7038) discloses that, when an extracellular matrix hydrogel (obtained by decellularization treatment of the pig-derived dermis or the pig-derived bladder, lyophilization and pepsin treatment) was applied to a defective part of the rat abdominal wall, the bladder-derived gel was decomposed more rapidly with a higher quantity of the muscle produced than the dermis-derived gel.

However, said hydrogel is not sufficiently well applied to a target site in a clinical situation and thus it is expected that the hydrogel is dropped out from the applied site or the matrix added is flown out and lost from the target site. A particulate decellularized tissue, depending on its type, is expected to remain in the applied site due to self-gelation but gelation is not completed instantaneously and affinity with a target site is not sufficient. In particular, in case of a dynamic tissue such as the heart, it is difficult to retain the hydrogel in the applied site and therefore it is suggested that a sufficient effect is not obtained.

Patent reference 2 (Japanese Patent Publication No. 2002-518319) reported that a particulate cell-free tissue matrix obtained after low-temperature disruption of AlloDerm (LifeCell), a decellularized tissue matrix of the human skin, when applied to rat or pig, showed no sign of severe acute inflammatory response.
Patent reference 1: Japanese Patent Publication No. 2013-42677
Patent reference 2: Japanese Patent Publication No. 2002-518319
Patent reference 3: Japanese Patent No. 4092397
Patent reference 4: Japanese Patent Re-Publication of WO2008/111530
Patent reference 5: Japanese Patent Publication No. 2009-50297
Patent reference 6: Japanese Patent Publication No. 2010-227246
Patent reference 7: Japanese Patent No. 4189484

Non-patent reference 1: Sasaki S., et al., Mol. Vis., 2009, 15, 2022-2028
Non-patent reference 2: Hashimoto Y., et al., Biomaterials, 2010, 31, 3941-3948
Non-patent reference 3: Funamoto S., et al., Biomaterials, 2010, 31, 3590-3595
Non-patent reference 4: Negishi J., et al., J. Artif. Organs, 2011, 14, 223-231
Non-patent reference 5: Singelyn J.M., et al., Biomaterials, 2009, 30, 5409-5416
Non-patent reference 6: Seif-Naraghi S., et al., J. Vis. Exp., 2010, 46, e2109
Non-patent reference 7: Singelyn J.M., et al., J. Am. Coll. Cardiol., 2012, 59, 751-763
Non-patent reference 8: Sonya B., et al., Sci. Transl. Med., 2013, 5, 173ra25
Non-patent reference 9: Wolf M.T., et al., Biomaterials, 2012, 33, 7028-7038
Non-patent reference 10: Ott H. C., et al., Nature Medicine, 2008, 14, 213-221
Non-patent reference 11: Barakat O., et al., J. Surg. Res., 2012, 173, e11-e25
Non-patent reference 12: Park K.M., et al., Transplant. Proc., 2012, 44, 1151-1154
Non-patent reference 13: Ott H. C., et al., Nature Medicine, 2010, 16, 927-933
Non-patent reference 14: Yang B., et al., Tissue Eng. Part C Methods, 2010, 16, 1201-1211
Non-patent reference 15: DeQuach J.A., et al., Tissue Eng. Part A, 2011, 17, 2583-2592

### DISCLOSURE OF THE INVENTION

### (Technical Problem to be Solved by the Invention)

As mentioned above, it has been shown that a particulate decellularized tissue does not induce rejection when used as a graft and is useful for regeneration and cure of affected sites. However, to make it better, the followings are desired: namely, providing a scaffold where assembling to target sites of cells from patients involved in regeneration and cure or of cells introduced, dedifferentiation of mature cells at target sites, growth, differentiation and redifferentiation of these cells take place rapidly; or tissues or organs reconstructed with a particulate decellularized tissue are rapidly adaptable to a host and are functional; being easily applicable to target sites and rapidly gelled and surely detained at the sites; in addition, being capable of inducing at an early stage microvasculature for supplying nutrient to assembled cells to promote the aforementioned effects and more effective tissue regeneration.

### (Means for Solving the Problems)

In order to solve the problems, the present inventors have earnestly studied and as a result have found that, when a particulate derived from a decellularized tissue is applied in combination with fibrin glue to cells or a disease model animal, (1) differentiation to myocardial cells and gain of functions such as beating were observed in case of cells from the heart; (2) the effect of inhibition to thinning of the heart wall and the effect of neovascularization were confirmed in a myocardial infarction model; (3) formation and induction of dendrites / outgrowth of neurites were observed in neural progenitor cells; and (4) the effect of acceleration of tissue regeneration while inhibiting contracture at a frostbite site was confirmed in a skin frostbite model, to thereby complete the present invention. Thus, the present invention includes the followings.
- A hybrid gel comprising a particulate decellularized tissue obtained by pulverizing animal-derived biological tissues that are decellularized (decellularized biological tissues), fibrinogen and thrombin, wherein thrombin is at a concentration of 0.8 U/mL to 125 U/mL and fibrinogen is at a concentration of 30 mg/mL to 40 mg/mL;
- A medicament for treating diseases, a cell transplantation adjuvant and a cell culture material comprising said hybrid gel;
- A method for preparing a hybrid gel comprising a step of decellularizing animal-derived biological tissues to give decellularized biological tissues, a step of pulverizing said decellularized biological tissues to give a particulate decellularized tissue, and a step of mixing said particulate decellularized tissue, fibrinogen and thrombin, wherein thrombin is at a concentration of 0.8 U/mL to 125 U/mL and fibrinogen is at a concentration of 30 mg/mL to 40 mg/mL;
- Also described herein is a therapy for tissue regeneration comprising applying said particulate decellularized tissue and fibrin glue to an animal tissue;
- Also described herein is a therapy for tissue regeneration comprising applying said hybrid gel to an animal tissue;
- Also described herein is a method for cell transplantation comprising applying said particulate decellularized tissue, fibrin glue and cells to an animal tissue;
- Also described herein is a method for cell transplantation comprising applying cells together with said hybrid gel to an animal tissue;
- Also described herein is a method for cell culture comprising culturing cells together with said hybrid gel;
- Also described herein is a kit comprising a particulate decellularized tissue obtained by pulverizing animal-derived biological tissues that are decellularized (decellularized biological tissues), and fibrin glue.

Specifically, the present invention includes the following:
[1] A hybrid gel comprising a particulate decellularized tissue obtained by pulverizing animal-derived biological tissues that are decellularized (decellularized biological tissues), fibrinogen and thrombin, wherein thrombin is at a concentration of 0.8 U/mL to 125 U/mL and fibrinogen is at a concentration of 30 mg/mL to 40 mg/mL.
[2] A method for preparing a hybrid gel comprising a step of decellularizing animal-derived biological tissues to give decellularized biological tissues, a step of pulverizing said decellularized biological tissues to give a particulate decellularized tissue, and a step of mixing said particulate decellularized tissue, fibrinogen and thrombin, wherein thrombin is at a concentration of 0.8 U/mL to 125 U/mL and fibrinogen is at a concentration of 30 mg/mL to 40 mg/mL.
[3] The hybrid gel according to [1] or the method of 2, wherein the biological tissues that are to be decellularized are the ones having a matrix structure.
[4] The hybrid gel according to [1] or [3] or the method according to [2] or [3], wherein the biological tissues that are to be decellularized are one or more of biological tissues selected from the group consisting of the heart, the liver, the kidney, the lung, the brain and the spiral cord.
[5] The hybrid gel according to any one of [1], [3] and [4] or the method according to any one of [2] to [4], wherein thrombin is at a concentration of 0.8 U/mL to 12.5 U/mL.
[6] The hybrid gel according to any one of [1] and [3] to [5], wherein the decellularized biological tissues are the ones prepared by treatment of biological tissues with a high hydrostatic pressure.
[7] The method according to any one of [2] to [5], wherein the decellularization is conducted by treatment of biological tissues with a high hydrostatic pressure.
[8] The method according to any one of [2] to [5] and [7], wherein the step giving decellularized biological tissues comprises a step of decellularizing animal-derived biological tissues and a step of microwave irradiation and washing.
[9] The method according to any one of [2] to [5], [7] and [8], wherein the step of pulverizing the decellularized biological tissues comprises a step of shredding the decellularized biological tissues, a step of lyophilizing or freezing the decellularized biological tissues as shredded, and a step of pulverizing the decellularized biological tissues as lyophilized or frozen.
[10] The method according to [9], wherein the method further comprises a step of sieving the decellularized biological tissues as pulverized based on a particle size.
[11] A medicament comprising the hybrid gel as set forth in any one of [1] and [3] to [6] for use in treating diseases.
[12] The medicament according to [11], wherein the medicament is for use in treating myocardial infarction, diseases caused by nerve damage or skin frostbite.
[13] A cell transplantation adjuvant or a cell culture material comprising the hybrid gel as set forth in any one of [1] and [3] to [6] .
[14] The hybrid gel according to any one of [1] and [3] to [6] for use in tissue regeneration of an animal tissue.
[15] The hybrid gel according to any one of [1] and [3] to [5], the method according to any one of [2] to [5] and [7] to [10], the hybrid gel for use of [14], or wherein the animal is an animal other than human.
[16] A method for cell culture comprising culturing cells together with the hybrid gel as set forth in [1] and [3] to [6].
[17] The cell transplantation adjuvant or cell culture material according to [13], or the method for cell culture according to [16], wherein the cells are the heart-derived cells, neural progenitor cells, or tissue stem cells.

### EFFECTS OF THE INVENTION

The hybrid gel of the present invention exerts the effect to promote differentiation and gain of function of stem cells and the therapeutic effect to a variety of diseases. More specifically, the effects exerted by the present invention includes: (1) the effect that the heart-derived cells are differentiated into myocardial cells and gains functions such as beating; (2) the effect of inhibition to thinning of the heart wall and the effect of neovascularization after myocardial infarction; (3) the effect that neural progenitor cells gain ability to form dendrites and ability to outgrow neurites; and (4) the effect of inhibiting contracture at a frostbite site and of accelerating tissue regeneration after skin frostbite.

### BRIEF DESCRIPTION OF DRAWINGS

Fig. 1 shows the results of no treatment in myocardial infarction model rat.
Fig. 2 shows the results of application of fibrin glue to myocardial infarction model rat.
Fig. 3 shows the results of application of fibrin glue to myocardial infarction model rat.
Fig. 4 shows the results of application of the hybrid gel to myocardial infarction model rat.
Fig. 5 shows the results of application of the hybrid gel to myocardial infarction model rat.
Fig. 6 shows the results of measurement of the number of neovascularization in an infarcted area.
Fig. 7 shows the results of estimation of the action to induce formation of dendrites in neural progenitor cells.
Fig. 8 shows the results of estimation of differentiation of neural progenitor cells into nerve cells.
Fig. 9 shows the results of estimation of ability to induce subcutaneous neovascularization.
Fig. 10 shows the results of estimation of skin tissue regeneration at a frostbite site.

### BEST MODE FOR CARRYING OUT THE INVENTION

### 1. Method for preparing hybrid gel

The present invention includes a method for preparing a hybrid gel comprising a step of decellularizing animal-derived biological tissues to give decellularized biological tissues, a step of pulverizing said decellularized biological tissues to give a particulate decellularized tissue, and a step of mixing said particulate decellularized tissue, fibrinogen and thrombin, wherein thrombin is at a concentration of 0.8 U/mL to 125 U/mL and fibrinogen is at a concentration of 30 mg/mL to 40 mg/mL.

In accordance with the method of the present invention, initially biological tissues are collected from animals. The biological tissues are decellularized to remove animal-derived cells, viruses and bacteria. Thus, even if the biological tissues are transplanted to animals different from those from which the biological tissues are obtained, a heterologous immune reaction is restrained. Therefore, the kind of animals from which biological tissues are collected is not particularly limited. On the other hand, since biological tissues are preferably readily available, they are preferably collected from an animal other than human, preferably domestic animals of mammals or domestic animals of birds. The domestic animals of mammals include cattle, horses, camels, llama, donkey, yak, sheep, pigs, goats, deer, alpacas, dogs, raccoon dogs, weasels, foxes, cats, rabbits, hamsters, guinea pigs, rats, mice, squirrels, raccoons, and the like. The domestic animals of birds include parakeet, parrot, chicken, ducks, turkeys, geese, guinea fowl, pheasant, ostrich, quail, and the like. Among these, biological tissues from pigs and rabbits are preferable in view of stable availability.

The biological tissues include tissues which have an extracellular matrix structure and which can be decellularized, the tissues being selected from the group consisting of liver, kidney, ureter, bladder, urethra, tongue, tonsils, esophagus, stomach, small intestine, large intestine, anus, pancreas, heart, blood vessels, spleen, lung, brain, bone, spinal cord, cartilage, testis, uterus, fallopian tube, ovary, placenta, cornea, skeletal muscle, tendons, nerves, skin, and the like, most preferably selected from the group consisting of heart, liver, kidney, lung, brain, spiral cord, testis, spleen, bladder, blood vessels, skin, and the like. Most preferable as biological tissues are heart, liver, kidney, lung, brain and spiral cord. Methods for collecting biological tissues from animals are known in the art.

Next, in accordance with the method of the present invention, biological tissues from animals are decellularized. Decellularization treatment is not particularly limited as long as it can remove animal-derived cells, virus and bacteria and includes treatment with a surfactant (Non-patent reference 5, Non-patent reference 7, Non-patent reference 8), enzyme treatment, osmotic pressure treatment, freeze and thawing, treatment with a high hydrostatic pressure (Non-patent references 1, 2, 3, 4, Patent reference 3: JP Patent 4092397, Patent reference 4: JP 2008-111530, Patent reference 5: JP 2009-50297), which may suitably be selected depending on the kind of animals and biological tissues. Treatment with a high hydrostatic pressure is particularly preferable in view of no use of chemicals that may affect adversely to the human body such as a surfactant. Treatment with a high hydrostatic pressure is done at a pressure of 2 to 1500 MPa, preferably 10 to 1000 MPa, more preferably 80 to 500 MPa.

The conditions for decellularization may suitably be selected depending on the kind of animals and biological tissues. The conditions for decellularization is known in the art and disclosed in Non-patent reference 10 (Ott H. C., et al., Nature Medicine, 2008, 14, 213-221) for the heart, Non-patent reference 11 (Barakat O., et al., J. Surg. Res., 2012, 173, e11-e25) for the liver, Non-patent reference 12 (Park K. M., et al., Transplant. Proc., 2012, 44, 1151-1154) for the stomach, the intestine, the spleen and the kidney, Non-patent reference 13 (Ott H. C., et al., Nature Medicine, 2010, 16, 927-933) for the lung, Non-patent reference 14 (Yang B., et al., Tissue Eng. Part C Methods, 2010, 16, 1201-1211) for the bladder, Non-patent references 3 and 4 for the blood vessel, Non-patent reference 15 (DeQuach J.A., et al., Tissue Eng. Part A, 2011, 17, 2583-2592) for the brain and Non-patent reference 9 for the skin.

Decellularization treatment may include a step of washing decellularized biological tissues. A method for washing decellularized biological tissues may suitably be selected depending on the kind of decellularization treatment. A method for washing includes immersing in a washing solution (Patent reference 6: JP 2010-227246) and irradiation of microwave (Patent reference 7: JP 4189484).

Next, in accordance with the method of the present invention, pulverization is conducted to the decellularized biological tissues. A step of pulverization may include a step of shredding the decellularized biological tissues, a step of lyophilizing or freezing the decellularized biological tissues as shredded, and a step of pulverizing the decellularized biological tissues as lyophilized or frozen. A method for preparing a particulate decellularized tissue is disclosed in Non-patent references 5, 6, 7 and 8 and Patent reference 2.

A method for pulverizing tissues is not particularly limited but includes pulverization of biological tissues at normal temperature, freezing biological tissues followed by pulverization under frozen conditions, and the like. For biological tissues which are difficult to be pulverized at normal temperature such as e.g. the kidney, pulverization under frozen conditions is preferable. In case of pulverization under frozen conditions, tissues are sometimes damaged due to ice crystal growth at around 0°C. Thus, the conditions of pulverization in case of pulverization under frozen conditions is -80°C to -5°C, preferably -50°C to -10°C, and more preferably -40°C to -15°C.

A method for pulverization includes ball mill, a bead mill, a colloid mill, a conical mill, disc mill, edge mills, milling mills, hammer mills, pellet mills, cutting mills, roller mills, jet mills, and the like, preferably cutting mills.

A step of pulverization may further include a step of sieving the decellularized biological tissues as pulverized based on a particle size.

A particle size of a particulate decellularized tissue is not particularly limited but is 0.1 to 1,000 µm, preferably 0.5 to 500 µm, and more preferably 1 to 100 µm since when the size is too small, the effect of tissue regeneration is low whereas when the size is too large, it becomes difficult to use the tissue as a material for transplant or treatment.

In accordance with the method for preparing a particulate decellularized tissue according to the present invention, decellularized tissues (or biological tissues) may be pulverized into particulates at any stage of from collecting biological tissues, decellularizing the biological tissues, washing and removing the destroyed cells, and obtaining a particulate decellularized tissue. Namely, regardless of whether a step of pulverization is conducted before or after decellularization, the obtained particulate decellularized tissues show no difference and can equally be used for the subsequent process for preparing a hybrid gel.

In accordance with the method for preparing a hybrid gel, a particulate decellularized tissue, fibrinogen and thrombin are mixed together to prepare a hybrid gel. A method for mixing includes mixing a mixture of a fibrinogen solution and a particulate decellularized tissue with a thrombin solution (or thrombin powder) and mixing a mixture of a thrombin solution and a particulate decellularized tissue with a fibrinogen solution (or fibrinogen powder).

A concentration of fibrinogen contained in a hybrid gel is not particularly limited as long as it can form a gel and may suitably be altered depending on the sites where a hybrid gel is applied or the usage of a hybrid gel. In accordance with the invention it is 30 mg/mL to 40 mg/mL.

A concentration of thrombin contained in a hybrid gel is not particularly limited as long as it can form a gel and may suitably be altered depending on the sites where a hybrid gel is applied or the usage of a hybrid gel. When a concentration of thrombin is 0.8 U/mL or more, a hybrid gel is readily formed. Thus, a concentration of thrombin may be 0.8 U/mL to 250 U/mL. On the other hand, when a concentration of thrombin in a gel is low, cellular adhesiveness was excellent. Therefore, in accordance with the invention the concentration of thrombin is0.8 U/mL to 125 U/mL, preferably 0.8 U/mL to 12.5 U/mL. However, since it is thought to be possible to prepare a hybrid gel by regulating time and temperature for solidification to gel even at a concentration of less than 0.8 U/mL of thrombin, a concentration of less than 0.8 U/mL may also be used.

A concentration of a particulate decellularized tissue contained in a hybrid gel may suitably be altered depending on the kind of particulate, the sites where a hybrid gel is applied or the usage of a hybrid gel. As an exemplary, it may be 16 µg/mL to 64 µg/mL.

### 2. Hybrid gel

The present invention includes a hybrid gel comprising a particulate decellularized tissue obtained by pulverizing animal-derived biological tissues that are decellularized tissues (decellularized biological tissues), fibrinogen and thrombin, wherein thrombin is at a concentration of 0.8 U/mL to 125 U/mL and fibrinogen is at a concentration of 30 mg/mL to 40 mg/mL.

As mentioned above, for the hybrid gel of the present invention, the kind of animals from which biological tissues are collected is not particularly limited. They are preferably collected from an animal other than human, preferably domestic animals of mammals or domestic animals of birds. The domestic animals of mammals include cattle, horses, camels, llama, donkey, yak, sheep, pigs, goats, deer, alpacas, dogs, raccoon dogs, weasels, foxes, cats, rabbits, hamsters, guinea pigs, rats, mice, squirrels, raccoons, and the like. The domestic animals of birds include parakeet, parrot, chicken, ducks, turkeys, geese, guinea fowl, pheasant, ostrich, quail, and the like. Among these, biological tissues from pigs and rabbits are preferable in view of stable availability.

The biological tissues include tissues which have an extracellular matrix structure and which can be decellularized, the tissues being selected from the group consisting of liver, kidney, ureter, bladder, urethra, tongue, tonsils, esophagus, stomach, small intestine, large intestine, anus, pancreas, heart, blood vessels, spleen, lung, brain, bone, spinal cord, cartilage, testis, uterus, fallopian tube, ovary, placenta, cornea, skeletal muscle, tendons, nerves, skin, and the like, most preferably selected from the group consisting of heart, liver, kidney, lung, brain, spiral cord, testis, spleen, bladder, blood vessels, skin, and the like. Most preferable as biological tissues are heart, liver, kidney, lung, brain and spiral cord.

As mentioned above, for the hybrid gel of the present invention, a method for decellularization is not particularly limited. It is preferably treatment with a high hydrostatic pressure.

As mentioned above, a concentration of fibrinogen in a hybrid gel is not particularly limited. In accordance with the invention it is 30 mg/mL to 40 mg/mL.

As mentioned above, a concentration of thrombin in a hybrid gel is not particularly limited as long as it can form a gel. As an exemplary, it may be 0.8 U/mL to 250 U/mL. In accordance with the invention it is 0.8 U/mL to 125 U/mL, preferably 0.8 U/mL to 12.5 U/mL. Also described herein is that a concentration of less than 0.8 U/mL may also be used.

As mentioned above, a concentration of a particulate in a hybrid gel is not particularly limited and may be 16 µg/mL to 64 µg/mL. A particle size of a particulate decellularized tissue is not particularly limited but is 0.1 to 1,000 µm, preferably 0.5 to 500 µm, and more preferably 1 to 100 µm.

### 3. Medicament for treating diseases

In accordance with the present invention, the hybrid gel as mentioned above proves to exert the effect to induce differentiation of the heart-derived cells, the effect of prognostic amelioration after myocardial infarction, the effect to induce differentiation of neural progenitor cells, and the effect of inhibiting contracture at a skin frostbite site and of curing the affected portion. Thus, the present invention includes a medicament for treating diseases comprising the hybrid gel as mentioned above. The hybrid gel may be under any of gelled condition, frozen condition or lyophilized condition. In case of the frozen condition, the hybrid gel may be thawed prior to application. In case of the lyophilization condition, the hybrid gel may be hydrated prior to application. The hybrid gel may directly be applied to the affected portion. The medicament for treating diseases of the present invention includes a medicament for treating myocardial infarction, a medicament for treating diseases caused by nerve damage and a medicament for treating skin frostbite.

### 4. Cell transplantation adjuvant and cell culture material

In accordance with the present invention, the hybrid gel proves to exert the effect of inducing differentiation of the heart-derived cells and of gaining beating function and the effect to induce differentiation of neural progenitor cells. Thus, the present invention includes a cell transplantation adjuvant and a cell culture material comprising the hybrid gel as mentioned above. A cell transplantation adjuvant either may primarily consist of the hybrid gel and cells to be transplanted or may primarily consist of the hybrid gel. In the former case, a cell transplantation adjuvant may be applied to animal tissues while cells to be transplanted are present in, or on the surface of, the hybrid gel. In the latter case, cells to be transplanted are combined with a cell transplantation adjuvant before or after application thereof to animal tissues.

A cell culture material may primarily consist of the hybrid gel and may be used with coculture with cells in a culture medium. For coexistence with cells, cells may directly be inoculated onto the hybrid gel or a cell culture insert may be set between the hybrid gel and cells.

Cells used in a cell transplantation adjuvant and a cell culture material are not particularly limited. Preferably, they are the heart-derived cells, neural progenitor cells, or tissue stem cells.

### 5. Therapy for tissue regeneration

Also described herein is a therapy for tissue regeneration comprising applying a particulate decellularized tissue obtained by pulverizing animal-derived biological tissues that are decellularized (decellularized biological tissues) and fibrin glue to an animal tissue. As described herein, a hybrid gel is formed on animal tissues by applying a particulate decellularized tissue and fibrin glue to the animal tissues. In this regard, the order of applying a particulate decellularized tissue and fibrin glue is not particularly limited. Also described herein is a therapy for tissue regeneration comprising applying the hybrid gel.

As mentioned above, for the therapy for tissue regeneration as described herein, the kind of animals from which biological tissues are collected is not particularly limited. They are preferably collected from an animal other than human, preferably domestic animals of mammals or domestic animals of birds. The domestic animals of mammals include cattle, horses, camels, llama, donkey, yak, sheep, pigs, goats, deer, alpacas, dogs, raccoon dogs, weasels, foxes, cats, rabbits, hamsters, guinea pigs, rats, mice, squirrels, raccoons, and the like. The domestic animals of birds include parakeet, parrot, chicken, ducks, turkeys, geese, guinea fowl, pheasant, ostrich, quail, and the like. Among these, biological tissues from pigs and rabbits are preferable in view of stable availability.

The biological tissues include tissues which have an extracellular matrix structure and which can be decellularized, the tissues being selected from the group consisting of liver, kidney, ureter, bladder, urethra, tongue, tonsils, esophagus, stomach, small intestine, large intestine, anus, pancreas, heart, blood vessels, spleen, lung, brain, bone, spinal cord, cartilage, testis, uterus, fallopian tube, ovary, placenta, cornea, skeletal muscle, tendons, nerves, skin, and the like, most preferably selected from the group consisting of heart, liver, kidney, lung, brain, spiral cord, testis, spleen, bladder, blood vessels, skin, and the like. Most preferable as biological tissues are heart, liver, kidney, lung, brain and spiral cord.

As mentioned above, for the therapy for tissue regeneration as describe herein, a method for decellularization is not particularly limited. It is preferably treatment with a high hydrostatic pressure.

Tissues to which the therapy for tissue regeneration of the present invention is applied are not particularly limited and are preferably the heart, the nerve and the skin.

The kind of fibrin glue as used herein is not particularly limited and preferably is Bolheal (manufactured by THE CHEMO-SERO-THERAPEUTIC RESEARCH INSTITUTE).

As mentioned above, a concentration of fibrinogen in a hybrid gel is not particularly limited. In accordance with the invention it is 30 mg/mL to 40 mg/mL.

As mentioned above, a concentration of thrombin in a hybrid gel is not particularly limited as long as it can form a gel. As an exemplary, it may be 0.8 U/mL to 250 U/mL. In accordance with the invention it is 0.8 U/mL to 125 U/mL, preferably 0.8 U/mL to 12.5 U/mL. On the other hand, a concentration of less than 0.8 U/mL is also described herein.

As mentioned above, a concentration of a particulate in a hybrid gel is not particularly limited and may be 16 µg/mL to 64 µg/mL. A particle size of a particulate decellularized tissue is not particularly limited but is 0.1 to 1,000 µm, preferably 0.5 to 500 µm, and more preferably 1 to 100 µm.

### 6. Method for cell transplantation

Also described herein is a method for cell transplantation comprising applying the particulate decellularized tissue, fibrin glue and cells to an animal tissue. In accordance with the method for cell transplantation, a hybrid gel is formed from the particulate decellularized tissue and fibrin glue and the cells are present in, or on the surface of, the hybrid gel. The order of applying the particulate decellularized tissue, fibrin glue and cells is not particularly limited. Each of these components may either be applied separately or simultaneously.

Also described herein is a method for cell transplantation comprising applying cells together with the hybrid gel as mentioned above to an animal tissue. The cells may be present in, or on the surface of, the hybrid gel. It may be either that the hybrid gel with the cells being therein or on the surface thereof is applied to an animal tissue or that one of either the hybrid gel or the cells is applied to an animal tissue and thereafter the other is applied. In the latter case, the order of applying the hybrid gel and the cells is not particularly limited.

Cells used for cell transplantation are not particularly limited. Preferably, they are the heart-derived cells, neural progenitor cells, or tissue stem cells. Tissues to which the method for cell transplantation is applied are not particularly limited and include the heart, the nerve and the skin.

### 7. Method for cell culture

The present invention includes a method for cell culture comprising culturing cells together with the hybrid gel as mentioned above. In accordance with the method for cell culture of the present invention, in case that both the hybrid gel and cells are present in a culture medium, cells may directly be inoculated onto the hybrid gel or a cell culture insert may be set between the hybrid gel and cells. Cells to be cultured are not particularly limited and include the heart-derived cells, neural progenitor cells, or tissue stem cells.

### 8. Kit

Also described herein is a kit comprising a particulate decellularized tissue obtained by pulverizing animal-derived biological tissues that are decellularized (decellularized biological tissues), and fibrin glue. In accordance with the kit, when the particulate decellularized tissue and fibrin glue are applied to an animal tissue, a hybrid gel is formed at the application site.

As mentioned above, for the kit described herein, the kind of animals from which biological tissues are collected is not particularly limited. They are preferably collected from an animal other than human, preferably domestic animals of mammals or domestic animals of birds. The domestic animals of mammals include cattle, horses, camels, llama, donkey, yak, sheep, pigs, goats, deer, alpacas, dogs, raccoon dogs, weasels, foxes, cats, rabbits, hamsters, guinea pigs, rats, mice, squirrels, raccoons, and the like. The domestic animals of birds include parakeet, parrot, chicken, ducks, turkeys, geese, guinea fowl, pheasant, ostrich, quail, and the like. Among these, biological tissues from pigs and rabbits are preferable in view of stable availability.

The biological tissues include tissues which have an extracellular matrix structure and which can be decellularized, the tissues being selected from the group consisting of liver, kidney, ureter, bladder, urethra, tongue, tonsils, esophagus, stomach, small intestine, large intestine, anus, pancreas, heart, blood vessels, spleen, lung, brain, bone, spinal cord, cartilage, testis, uterus, fallopian tube, ovary, placenta, cornea, skeletal muscle, tendons, nerves, skin, and the like, most preferably selected from the group consisting of heart, liver, kidney, lung, brain, spiral cord, testis, spleen, bladder, blood vessels, skin, and the like. Most preferable as biological tissues are heart, liver, kidney, lung, brain and spiral cord.

As mentioned above, for the hybrid gel described herein, a method for decellularization is not particularly limited. It is preferably treatment with a high hydrostatic pressure. A particle size of a particulate decellularized tissue is not particularly limited but is 0.1 to 1,000 µm, preferably 0.5 to 500 µm, and more preferably 1 to 100 µm.

As is described herein, the hybrid gel as mentioned above may exert excellent effects in a disease model (myocardial infarction model and skin frostbite model) and cells (heart-derived cells and neural progenitor cells). Thus, the kit described herein includes a kit for treating diseases. Such a kit for treating diseases includes a kit for treating myocardial infarction, a kit for treating diseases caused by nerve damage and a kit for treating skin frostbite. Also, the kit described herein includes a kit for cell transplantation and a kit for cell culture. The kind of cells is not particularly limited and includes the heart-derived cells, neural progenitor cells, or tissue stem cells.

### [Examples]

The present invention is explained in more detail by means of the following Examples but is not limited thereto.

### Example 1

### Investigation on effect of particulate decellularized tissue from heart of adult rabbit on culture of rabbit fetus heart-derived cells

### 1. Preparation of rabbit fetus heart-derived cells

To Japanese White female rabbit of about 6 months old (Oriental Yeast Co.,Ltd.) was subcutaneously injected FSH (anterior follicle stimulating hormone: Antolin R · 10, Kyoritsu Seiyaku Corporation) at 0.5 U/animal for six times with 12 hour interval. About five hours after the final injection of FSH, the rabbit was intravenously administered with hCG (chorionic gonadotrophin: Puberogen, Novartis) at 50 U/animal and mated with a male rabbit to prepare a pregnant rabbit. On Day 16.5 to 18.5 after mating, the pregnant rabbit was euthanatized by intravenous administration of about 200 mg of pentobarbital injection (Somnopentyl, Kyoritsu Seiyaku Corporation). After laparotomy, a fetus was taken from the uterine and was suspended in saline (Otsuka normal saline, Otsuka Pharmaceutical Co., Ltd.). The heart was removed from the fetus and dispersed in a trypsin solution of about 1.25% (Difco) to prepare the cells.

### 2. Preparation of particulate decellularized tissue

The heart was removed from Japanese White rabbit of about 6 months to 12 months old (Oriental Yeast Co.,Ltd.) and was washed with saline. After washing, the tissue was put in a polyethylene bag with saline and the bag was sealed. With Dr.Chef (Kobe Steel, Ltd.), a high hydrostatic pressure treatment was conducted at 3,000 to 10,000 atm. The tissues after the high hydrostatic pressure treatment were washed with a washing solution containing a nuclease and a washing solution containing an alcohol. After the washing was completed, each of the decellularized tissues was lyophilized with a lyophilizer (Eyela Corporation). The lyophilized decellularized tissues were pulverized with a mill, a food processor, an agate pulverizer (AS ONE Corporation) and the like. The particulate decellularized tissues were sieved to prepare particulate decellularized tissues with a diameter of 500 µm or less. The particulate decellularized tissues were stored at 4°C till use and were used at 2 mg/mL with saline.

### 3. Culture

To a TERASAKI plate (Sumiron Co., Ltd.) coated with 0.1% gelatin (Sigma) were added 10 µL per well of Bolheal A solution [prepared in accordance with an attached document; containing a final concentration (in Bolheal) of 100 ng/mL of G-CSF (Diaclone), 10 ng/mL of bFGF (PROGEN) and 7 µg/mL of selenoprotein P fragment (prepared in-house)] supplemented with the particulate decellularized tissue from the heart of adult rabbit or about 10⁵ per well of the rabbit fetus heart-derived cells and then 10 µL per well of Bolheal B solution [prepared in accordance with an attached document and diluted to 1/10 with saline] for coagulation (fibrin formation). After coagulation, a culture medium (DMEM (Sigma) containing 10% FBS (Funakoshi Co., Ltd.) was overlay for culture in CO₂ incubator (5%, 37°C).

### 4. Group constitution and observation

For three groups, i.e. adult rabbit heart 16 (the particulate decellularized tissue from the heart of adult rabbit was added at a final concentration of 16 µg/mL), adult rabbit heart 64 (the particulate decellularized tissue from the heart of adult rabbit was added at a final concentration of 64 µg/mL) and No addition (no addition (0 µg/mL) of the particulate decellularized tissue), growth [qualitatively judged with criteria: death or no growth: -, growth by 1- to 2-fold: ±, growth by 2- to 5-fold: +, growth by 5-fold or more: ++], differentiation [judged from a change in shape from that when inoculated (spherical) (a change of a cell is maturation or maturation process to a differentiated cell), None: -, at least shape change is observed from spherical: ±, apparent shape change (cylindrical, flat, cell protrusion formation etc.) is observed: +, apparent differentiation (at a level where differentiation to a specific cell type is presumed such as heart muscle, fibroblast, complication of cell protrusion, etc.) is observed: ++] and beating [heartbeat, Not observed: -, at least one case is observed: ±, three or more cases are observed: +, many (six or more cases) are observed: ++] were estimated at Day 6 after culture.

### 5. Results

The results are shown in Table 1. For the rabbit fetus heart-derived cells cultured with no addition of the particulate decellularized tissue, growth was observed but not much differentiation was observed and pulsating cells were scarcely observed. When the particulate decellularized tissue was added, growth and differentiation were observed as well as beating of the cells. Besides, when the particulate decellularized tissue was added at 64 µg/mL, both differentiation and beating were prominent. As a result, it is apparent that the particulate decellularized tissue from the heart of adult rabbit contributes to differentiation into myocardial cells and gains functions such as beating of the rabbit fetus heart-derived cells.

**Table 1**

| Test groups | Conditions | | Results | | |
|---|---|---|---|---|---|
| | Bolheal | Particulate | Growth | Differentiation | Beating |
| Adult rabbit heart 16 | + | + | + | + | + |
| Adult rabbit heart 64 | + | + | + | + | ++ |
| No addition | + | - | + | ± | ± |

### Example 2

### Investigation on effect of particulate decellularized tissue of various kinds on cultured rabbit fetus heart-derived cells

### 1. Preparation of rabbit fetus heart-derived cells

The cells were obtained with the same procedures as described in Example 1.

### 2. Preparation of particulate decellularized tissue

The rabbit fetus heart obtained with the same procedures as described in Example 1, the heart, the kidney, the lung and the liver removed from Japanese White rabbit of about 6 months to 12 months old (Oriental Yeast Co.,Ltd.) and the liver obtained from pig were put in a polyethylene bag with saline and the bag was sealed. With Dr.Chef (Kobe Steel, Ltd.), a high hydrostatic pressure treatment was conducted at 3,000 to 10,000 atm. The tissues after the high hydrostatic pressure treatment were washed with a washing solution containing a nuclease and a washing solution containing an alcohol. After the washing was completed, each of the decellularized tissues was lyophilized with a lyophilizer (Eyela Corporation). The lyophilized decellularized tissues were pulverized with a mill, a food processor, an agate pulverizer (AS ONE Corporation) and the like. The particulate decellularized tissues were sieved to prepare particulate decellularized tissues with a diameter of 500 µm or less. The particulate decellularized tissues were stored at 4°C till use and were used at 2 mg/mL with saline.

### 3. Culture

To a Multidish 4 wells (Nunc) coated with 0.1% gelatin (Sigma) were added 10 µL per well of Bolheal A solution [prepared in accordance with an attached document and diluted to 1/2 with saline] supplemented with about 4 x 10⁵ per well of the rabbit fetus heart-derived cells and the particulate decellularized tissue as described above, respectively, and then 10 µL per well of Bolheal B solution [prepared in accordance with an attached document and diluted to 1/10 with saline] for coagulation (fibrin formation). After coagulation, a culture medium (DMEM (Sigma) containing 10% FBS (Funakoshi Co., Ltd.) was overlay for culture in CO₂ incubator (5%, 37°C).

### 4. Group constitution and observation

Examined were test groups where each 500 µg/mL of the particulate decellularized tissue from the rabbit fetus heart, the adult rabbit heart, the adult rabbit kidney, the adult rabbit lung, the adult rabbit liver and the adult pig liver was added, No addition (no addition (0 µg/mL) of the particulate decellularized tissue) and Control (no addition (0 µg/mL) of the particulate decellularized tissue/no addition of Bolheal (with inclusion)). As described in Example 1, growth, differentiation and beating were estimated at Day 7 after culture.

### 5. Results

The results are shown in Table 2. For Control, growth was not so much observed and tendency to differentiation into swelled cells was observed. Beating cells were not observed. For No addition group, growth/differentiation was observed but pulsating cells were not so much observed. To the contrary, for the groups where the particulate decellularized tissue was added, growth/differentiation was observed and pulsating cells were also observed irrespective of the kind of animals and the source of the organs. As a result, it is apparent that the particulate decellularized tissue, irrespective of its source, contributes to differentiation into myocardial cells and gains functions such as beating of the rabbit fetus heart-derived cells.

**Table 2**

| Test groups | Conditions | | Results | | |
|---|---|---|---|---|---|
| | Particulate | Bolheal | Growth | Differentiation | Beating |
| Rabbit fetus heart | + | + | + | + or ++ | + |
| Adult rabbit heart | + | + | + | + | + |
| Adult rabbit kidney | + | + | + | ++ | + |
| Adult rabbit lung | + | + | + | + | + |
| Adult rabbit liver | + | + | + | + | + |
| Adult pig liver | + | + | + | + | + |
| No addition | - | + | + | + | ± |
| Control | - | - | ± | + | - |

### Example 3

### Investigation on effect of hybrid gel of particulate decellularized tissue in rat myocardial infarction model

### 1. Preparation of particulate decellularized tissue

The liver tissue was removed from Wistar rat and washed with saline. After washing, the tissue was put in a polyethylene bag with saline and the bag was sealed. With Dr.Chef (Kobe Steel, Ltd.), a high hydrostatic pressure treatment was conducted at 3,000 to 10,000 atm. The tissues after the high hydrostatic pressure treatment were washed with a washing solution containing a nuclease and a washing solution containing an alcohol. After the washing was completed, each of the decellularized tissues was lyophilized with a lyophilizer (Eyela Corporation). The lyophilized decellularized tissues were pulverized with a mill, a food processor, an agate pulverizer (AS ONE Corporation) and the like. The particulate decellularized tissues were sieved and those with a diameter of 500 µm or less were used as particulate decellularized tissues.

### 2. Transplantation experiment

Wistar rat (male, 10-12 weeks old) was anesthetized by intramuscular injection of 0.1 mL of Somnopentyl. The side chest was shaved and disinfected with Isodine. A tube was inserted to the trachea of the rat under anesthesia and connected to a ventilator. The rat was laid in a side-lying position and incision was made from the side chest towards the heart, removing the muscle layer and the pericardium. The left coronary artery was ligated with surgical suture to create infarction. To the created infarction was added dropwise 100 µL of a fibrinogen solution (x1; 80 mg/mL) containing 10% of the particulate decellularized tissue from the liver and then was added dropwise 100 µL of a thrombin solution (x1; 250 U/mL, xl/150; 1.6 U/mL). As a control, group with dropwise addition of Bolheal alone and untreated group were used. The incised part of the rat, which underwent creation of infarction and various treatments, was sutured and, after spontaneous respiration became stable, the tube was removed.

### 3. Group constitution and observation

A concentration of fibrinogen in the hybrid gel was set to 40 mg/mL, a concentration of thrombin in the hybrid gel was set to 0.8 U/mL and 125 U/mL, and to the respective concentrations was added the particulate decellularized tissue at 10% of the gel. For comparison, group with no addition of the particulate decellularized tissue was also included. After 4 weeks, the rat was euthanatized, the infarction of the heart was observed and samples were collected. The collected samples were histologically estimated by HE stain for evaluation of the respective test groups.

### 4. Results

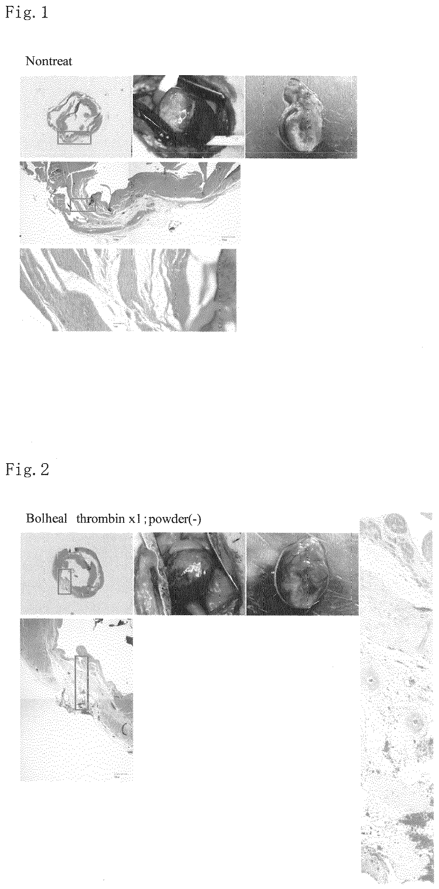

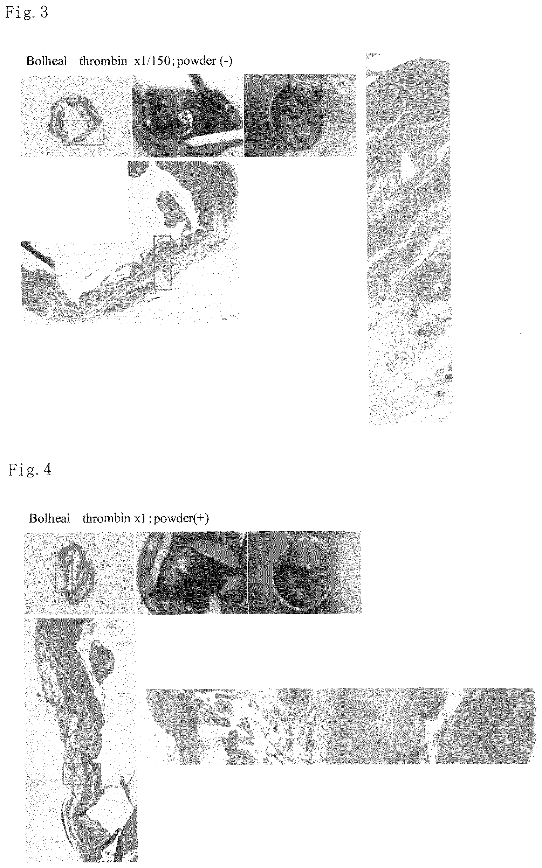

The results are shown in Fig. 1 to Fig. 5. Infiltration of neovascularization could be seen macroscopically even in the fibrin gel with no addition of the particulate decellularized tissue. With the thrombin concentration of 125 U/mL, neovascularization was inclined to be fewer. On the other hand, for the hybrid gel added with the particulate decellularized tissue, macroscopic observation revealed rich neovascularization in the surrounding and a lot of neovascularization into the infarcted heart muscle. In addition, an extent of neovascularization varied depending on a concentration of thrombin with higher neovascularization for the thrombin concentration of 0.8 U/mL.

### Example 4

### Investigation on effect of particulate decellularized tissue of various kinds on cultured rabbit fetus heart-derived cells

### 1. Preparation of rabbit fetus heart-derived cells

The cells were obtained with the same procedures as described in Example 1.

### 2. Preparation of particulate decellularized tissue

The tissue of the heart, the spleen, the brain, the spinal cord, the kidney and the bladder was removed from pig for food and washed with saline. After washing, the tissue was put in a polyethylene bag with saline and the bag was sealed. With Dr.Chef (Kobe Steel, Ltd.), a high hydrostatic pressure treatment was conducted at 3,000 to 10,000 atm. The tissues after the high hydrostatic pressure treatment were washed with a washing solution containing a nuclease and a washing solution containing an alcohol. After the washing was completed, each of the decellularized tissues was lyophilized with a lyophilizer (Eyela Corporation). The lyophilized decellularized tissues were pulverized with a mill, a food processor, an agate pulverizer (AS ONE Corporation) and the like. The particulate decellularized tissues were sieved to prepare particulate decellularized tissues with a diameter of 500 µm or less. The particulate decellularized tissues were stored at 4°C till use and were used at 2 mg/mL with saline.

### 3. Culture

The culture was conducted with the same procedures as described in Example 1.

### 4. Group constitution and observation

Examined were test groups where each 100 µg/mL of the particulate decellularized tissue of various kinds was added, No addition (no addition (0 µg/mL) of the particulate decellularized tissue), Positive control (with addition (100 µg/mL) of the particulate decellularized tissue from the heart of adult rabbit) and Control (no addition (0 µg/mL) of the particulate decellularized tissue/no addition of Bolheal (with inclusion)). As described in Example 1, beating was estimated at Day 7 after culture.

### 5. Results

The results are shown in Table 3. For Control, no beating was observed. For No addition group, pulsating cells were not so much observed. For the group where the particulate decellularized tissue from the rabbit heart was added, beating was observed for many cells. On the other hand, for the group where the particulate decellularized tissue of various kinds from the pig was added, pulsating cells were observed irrespective of the source of the organs. As a result, it is apparent that the particulate decellularized tissue, even if its source is heterologous, contributes to differentiation into myocardial cells and gains functions such as beating of the fetus heart-derived cells.

**Table 3**

| Test groups | Conditions | | Results (Beating) |
|---|---|---|---|
| | Particulate | Bolheal | |
| Rabbit heart decellularization | + | + | +++ |
| Pig heart decellularization | + | + | +++ |
| Pig spleen decellularization | + | + | ++ |
| Pig brain decellularization | + | + | +++ |
| Pig bone marrow decellularization | + | + | +++ |
| Pig kidney decellularization | + | + | +++ |
| Pig bladder decellularization | + | + | +++ |
| No addition | - | + | + |
| Control | - | - | - |

### Example 5

### Investigation on effect of hybrid gel of particulate decellularized tissue in rabbit myocardial infarction model

### 1. Preparation of particulate decellularized tissue

The particulate decellularized tissue was obtained with the same procedures as described in Example 2.

### 2. Transplantation experiment

Thoracotomy was performed to Japanese White rabbit (male, 14-15 weeks old when received, Biotek. Co.,Ltd.) under general anesthesia with ketamine and isoflurane on controlled artificial ventilation and the anterior interventricular branch of left coronary artery was ligated with surgical suture to create infarction. For group of the hybrid gel, about 40 µL of a fibrinogen solution was rubbed into the created infarction. A mixture of 5 µL of a solution in which 0.01 mg of the particulate decellularized tissue powder from rabbit fetus heart was suspended in saline and 5 µL of a fibrinogen solution was added dropwise, 10 µL of a thrombin solution (x 1/10: 25 U/mL) was added dropwise, and after 1 minute, a mixture of 100 µL of the fibrinogen solution and 100 µL of the thrombin solution was sprayed, which was left to stand for 1 minute. As a control, no transplantation group where creation of infarction alone was conducted was included. After all the procedures of transplantation were completed, the chest was closed.

### 3. Results

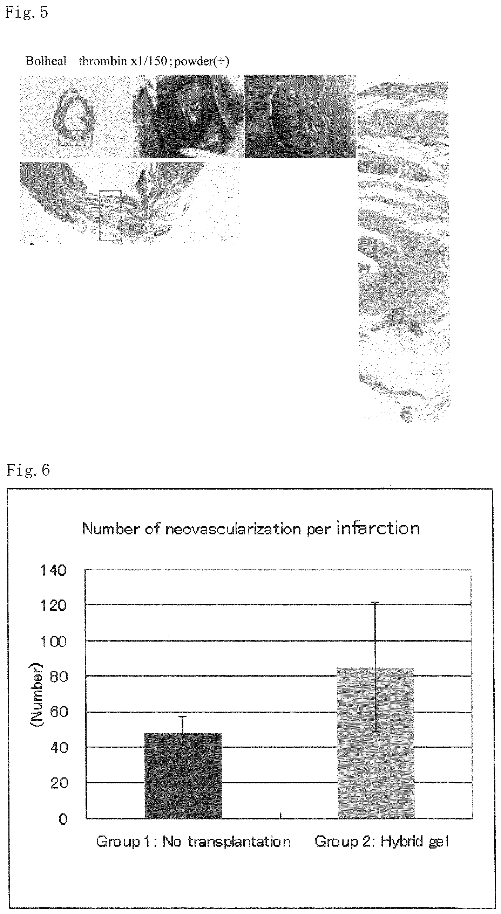

The results are shown in Fig. 6. In histopathological examination (hematoxylin-eosin stained slide specimens) after 2 months of the operation, more neovascularization at the infarction was observed in the group of the hybrid gel of the particulate decellularized tissue of the present invention than in no transplantation group.

### Example 6

### Investigation on effect of hybrid gel of particulate decellularized tissue of various kinds on neural progenitor cells

### 1. Preparation of particulate decellularized tissue of various kinds

The tissue of the brain, the spinal cord and the liver was removed from Wistar rat and washed with saline. After washing, the tissue was put in a polyethylene bag with saline and the bag was sealed. With Dr.Chef (Kobe Steel, Ltd.), a high hydrostatic pressure treatment was conducted at 3,000 to 10,000 atm. The tissues after the high hydrostatic pressure treatment were washed with a washing solution containing a nuclease and a washing solution containing an alcohol. After the washing was completed, each of the decellularized tissues was lyophilized with a lyophilizer (Eyela Corporation). The lyophilized decellularized tissues were pulverized with a mill, a food processor, an agate pulverizer (AS ONE Corporation) and the like. The particulate decellularized tissues were sieved to prepare particulate decellularized tissues with a diameter of 500 µm or less.

### 2. Culture and group constitution

For the respective particulate decellularized tissues from the brain, the spinal cord and the liver, group of culture on Bolheal containing the particulate decellularized tissues (on) and group of insert of Bolheal containing the particulate decellularized tissues (insert; Bolheal gel containing the particulate decellularized tissues was added onto cell culture insert) were examined.

For the group of culture on Bolheal containing the particulate decellularized tissues (on), to a 24-well plate dish was added dropwise 150 µl of a fibrinogen solution containing 10% particulate decellularized tissue and then was added dropwise 150 µl of a thrombin solution of various dilutions (x1; 250 U/mL, x1/10; 25 U/mL, x1/100; 2.5 U/mL) for mixture. PC12 cells (cells from rat adrenal pheochromocytoma) were inoculated onto the gel (1.0 x 10⁴ cells/well, 0.1% horse serum in RPMI) and cultured for 24 hours.

For the group of insert of Bolheal containing the particulate decellularized tissues (insert), PC12 cells (1.0 x 10⁴ cells/well, 10% horse serum · 5% FBS in RPMI) were inoculated to a 24-well plate dish coated with collagen and cultured for 24 hours. To cell culture insert (pore size 8 µm) was added dropwise 150 µl of a fibrinogen solution containing 10% particulate decellularized tissue and then was added dropwise 150 µl of a thrombin solution of various dilutions (x1; 250 U/mL, xl/10; 25 U/mL, x1/100; 2.5 U/mL) to prepare a gel containing the particulate decellularized tissues. The culture was exchanged with 0.1% horse serum in RPMI, the cell culture insert in which the gel was prepared was set on each of the well and the cells were cultured for 24 hours.

In respective investigation, negative control group with no addition of the particulate decellularized tissue and positive control group where 50 ng of NGF was added in place of the particulate decellularized tissue were included to verify that the estimation system functions.

### 3. Results

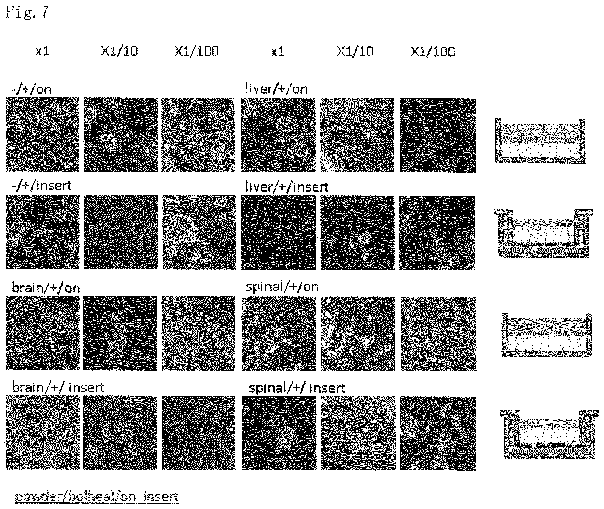

The results are shown in Fig. 7. As compared to the group where the particulate decellularized tissue from the liver was added, the group where the particulate decellularized tissue from the brain was added and the group where the particulate decellularized tissue from the spinal cord was added induced more formation of dendrites equivalent to that of NGF. In addition, it was observed that a lower concentration of thrombin showed higher formation of dendrites.

### Example 7

### Investigation on effect of hybrid gel of heterologous particulate decellularized tissue on neural progenitor cells

### 1. Preparation of particulate decellularized tissue

The tissue of the brain was removed from pig for food and washed with saline. After washing, the tissue was put in a polyethylene bag with saline and the bag was sealed. With Dr.Chef (Kobe Steel, Ltd.), a high hydrostatic pressure treatment was conducted at 3,000 to 10,000 atm. The tissues after the high hydrostatic pressure treatment were washed with a washing solution containing a nuclease and a washing solution containing an alcohol. After the washing was completed, each of the decellularized tissues was lyophilized with a lyophilizer (Eyela Corporation). The lyophilized decellularized tissues were pulverized with a mill, a food processor, an agate pulverizer (AS ONE Corporation) and the like. The particulate decellularized tissues were sieved to prepare particulate decellularized tissues with a diameter of 500 µm or less.

### 2. Culture and group constitution

For the particulate decellularized tissues from the pig brain, group wherein Bolheal gel with powder inclusion therein is added and group wherein Bolheal gel is added were examined. To a 96-well plate coated with collagen IV were inoculated PC12 cells at 1 x 10⁴ cells/100 µL (10% horse serum-5% FBS-RPMI1640)/well. After 24 hours, the culture medium was exchanged with 0.1% horse serum-RPMI1640 (100 µL/well) and the particulate decellularized tissues, Bolheal and reagents were added depending on the constitution of the groups. For the group wherein Bolheal gel with powder inclusion therein is added, using a TERASAKI plate (Sumiron Co., Ltd.), to 10 µL of a thrombin solution at 2.5 U/mL was added 0.3 mg/5 µL of the particulate decellularized tissues from the pig brain and then was added 5 µL of a fibrinogen solution. The solutions were mixed by pipetting to cause gelation (more than 2 hours at 37°C) and were added to the wells. For the group wherein Bolheal gel is added, using a TERASAKI plate, 10 µL of a thrombin solution at 2.5 U/mL, 5 µL of saline (Otsuka Pharmaceutical Co., Ltd.) and 5 µL of a fibrinogen solution were added sequentially. The solutions were mixed by pipetting to cause gelation (more than 2 hours at 37°C) and were added to the wells. In addition, positive control group where NGF is added at 50ng/mL and negative control group with no treatment were included. Two to three days after the treatment, induction of nerve cell differentiation was analyzed with neurite outgrowth and detection of a nerve cell-specific antigen as an index. Detection of a nerve cell-specific antigen was performed by immunohistochemical staining. For immunohistochemical staining, Anti-βIII Tubulin (Promega) as an antibody for detection of the antigen and Alexa Fluor 594 anti-mouse (Invitrogen) as an antibody for visualization were used. Hoechst 33342 (DOJINDO) was used for nuclear stain. With the above staining conditions, the nucleus of cells was stained blue whereas βIII Tubulin was stained red.

### 3. Results

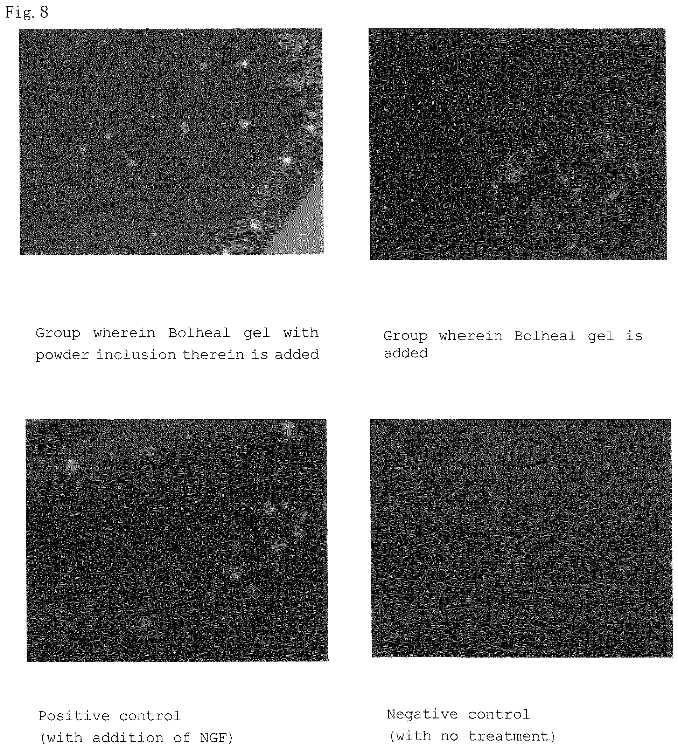

The results obtained two days after culture by observation of neurite outgrowth are shown in Table 4. Neurite outgrowth was observed in the group wherein Bolheal gel with powder inclusion therein is added and the Positive control group (with addition of NGF). The results obtained three days after culture by immunohistological observation are shown in Fig. 8 and Table 5. Immunohistochemical staining also revealed βIII Tubulin antigen in the group wherein Bolheal gel with powder inclusion therein is added and the Positive control group (with addition of NGF). As a result, nerve differentiation was confirmed in the group wherein Bolheal gel with powder inclusion therein is added to prove that the hybrid gel even with the particulate decellularized tissues from heterologous animals could induce nerve cells.

**Table 4**

| Test groups | Conditions | | Results (Neurite outgrowth) |
|---|---|---|---|
| | Particulate | Bolheal | |
| Group wherein Bolheal gel with powder inclusion therein is added | + | + | + |
| Group wherein Bolheal gel is added | - | + | - |
| Positive control (with addition of NGF) | - | - | + |
| Negative control (with no treatment) | - | - | - |

**Table 5**

| Groups | Positive ratio (%) * |
|---|---|
| Group wherein Bolheal gel with powder inclusion therein is added | 86 |
| Group wherein Bolheal gel is added | 0 |
| Positive control (with addition of NGF) | 92 |
| Negative control (with no treatment) | 0 |

| | |
|---|---|
| *Positive ratio = Number of cells stained with βIII Tubulin/Counted number of cells (Counted number of cells >20) x 100 | |

### Example 8

### Investigation on effect of hybrid gel of particulate decellularized tissue in rat back subcutaneously

### 1. Preparation of particulate decellularized tissue

The liver was removed from Wistar rat and washed with saline. After washing, the tissue was put in a polyethylene bag with saline and the bag was sealed. With Dr.Chef (Kobe Steel, Ltd.), a high hydrostatic pressure treatment was conducted at 3,000 to 10,000 atm. The tissues after the high hydrostatic pressure treatment were washed with a washing solution containing a nuclease and a washing solution containing an alcohol. After the washing was completed, each of the decellularized tissues was lyophilized with a lyophilizer (Eyela Corporation). The lyophilized decellularized tissues were pulverized with a mill, a food processor, an agate pulverizer (AS ONE Corporation) and the like. The particulate decellularized tissues were sieved and those with a diameter of 500 µm or less were used as particulate decellularized tissues.

### 2. Transplantation experiment

Wistar rat (male, 8-12 weeks old) was anesthetized by intramuscular injection of 0.1 mL of Somnopentyl. The back was shaved and disinfected with Isodine. Incision of 1 cm was created in the disinfected back and therefrom a pocket for inserting the hybrid gel was formed between the skin and the muscular layer. The hybrid gel consisting of fibrin gel was transplanted into the pocket as formed and the incision was ligated with 5-0 surgical suture. After a given period, the rat was euthanatized, the wound was observed and samples were collected. The collected samples were histologically estimated by HE stain.

### 3. Group constitution and observation

A concentration of fibrinogen in the hybrid gel was set to 40 mg/mL, a concentration of thrombin in the hybrid gel was set to 0.8 U/mL and 125 U/mL, and to the respective concentrations was added the particulate decellularized tissue at 5% of the gel. For comparison, group with no addition of the particulate decellularized tissue was also included and the respective test groups were evaluated.

### 4. Results

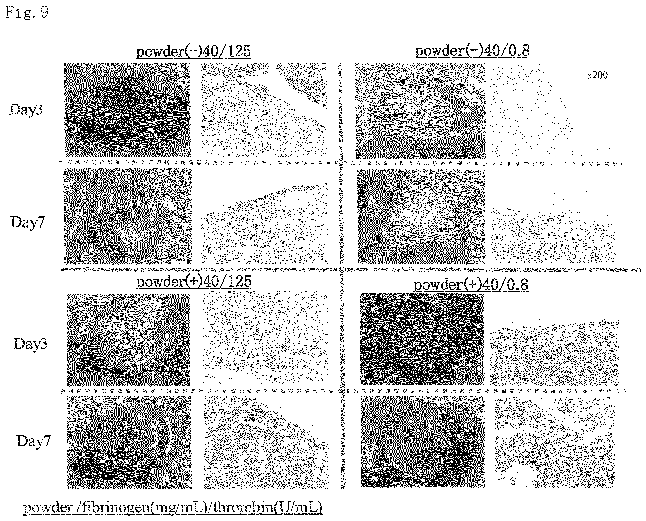

The results are shown in Fig. 9. Infiltration of neovascularization could be seen macroscopically even in the fibrin gel with no addition of the particulate decellularized tissue. Irrespective of a thrombin concentration, cellular infiltration could hardly be seen. No significant difference could be seen between Day 3 and Day 7 after transplantation. On the other hand, for the hybrid gel added with the particulate decellularized tissue, macroscopic observation revealed rich neovascularization in the surrounding and cellular infiltration and neovascularization into the interior of the gel. In addition, its extent varied depending on a concentration of thrombin with higher cellular infiltration and neovascularization for the thrombin concentration of 0.8 U/mL.

### Example 9

### Investigation on effect of hybrid gel of particulate decellularized tissue in rat skin frostbite model

### 1. Preparation of particulate decellularized tissue

The liver was removed from Wistar rat and washed with saline. After washing, the tissue was put in a polyethylene bag with saline and the bag was sealed. With Dr.Chef (Kobe Steel, Ltd.), a high hydrostatic pressure treatment was conducted at 3,000 to 10,000 atm. The tissues after the high hydrostatic pressure treatment were washed with a washing solution containing a nuclease and a washing solution containing an alcohol. After the washing was completed, each of the decellularized tissues was lyophilized with a lyophilizer (Eyela Corporation). The lyophilized decellularized tissues were pulverized with a mill, a food processor, an agate pulverizer (AS ONE Corporation) and the like. The particulate decellularized tissues were sieved and those with a diameter of 500 µm or less were used as particulate decellularized tissues.

### 2. Transplantation experiment

Wistar rat (male, 8-12 weeks old) was anesthetized by intramuscular injection of 0.1 mL of Somnopentyl. The back was shaved and disinfected with Isodine. Deficiency to the mid-dermal layer (diameter 15 mm) was created in the disinfected back. For creation of frostbite, a metallic body cooled with liquid nitrogen was pressed to the wound for 60 seconds. To the wound of frostbite was added the particulate decellularized tissue (liver), was added dropwise 80 mg/mL of a fibrin solution, and was added dropwise 250 U/mL of a thrombin solution. As a control, group with addition of Bolheal alone and group with no treatment were included. Then, the wound region was covered with an adhesive plaster, and the whole circumference of the trunk of the rat was covered with a gauze.

### 3. Group constitution and observation

A concentration of fibrinogen in the hybrid gel was set to 40 mg/mL, a concentration of thrombin in the hybrid gel was set to 125 U/mL, and to the respective concentrations was added the particulate decellularized tissue at 10% of the gel. For comparison, group with no addition of the particulate decellularized tissue was also included and, after a given period, the wound was observed.

### 4. Results

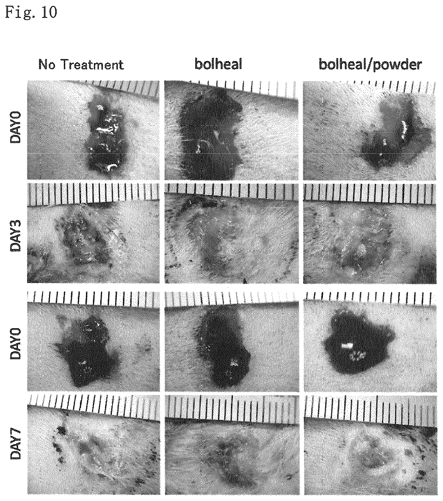

The results are shown in Fig. 10. In the group with no treatment, contracture of the skin peculiar to the curing process of the skin tissue was observed at a frostbite site. On the hand, in the group with no addition of the particulate decellularized tissue, no contracture of the skin could be seen. However, it stopped short of achieving filling of the skin deficiency due to frostbite but brought about fragile tissues covering the frostbite site. In the group with addition of the particulate decellularized tissue, no contracture of the skin could be seen and macroscopically there was evidence that tissue regeneration of the skin deficiency occurred early. As a result, the decellularized powder was thought to a material that promotes cell assembly, which accelerates skin regeneration, and filling of scaffold.

## Claims

1. A hybrid gel comprising a particulate decellularized tissue obtained by pulverizing animal-derived biological tissues that are decellularized (decellularized biological tissues), fibrinogen and thrombin, wherein thrombin is at a concentration of 0.8 U/mL to 125 U/mL and fibrinogen is at a concentration of 30 mg/mL to 40 mg/mL.

2. A method for preparing a hybrid gel comprising a step of decellularizing animal-derived biological tissues to give decellularized biological tissues, a step of pulverizing said decellularized biological tissues to give a particulate decellularized tissue, and a step of mixing said particulate decellularized tissue, fibrinogen and thrombin, wherein thrombin is at a concentration of 0.8 U/mL to 125 U/mL and fibrinogen is at a concentration of 30 mg/mL to 40 mg/mL.

3. The hybrid gel according to claim 1 or the method of claim 2, wherein the biological tissues that are to be decellularized are the ones having a matrix structure.

4. The hybrid gel according to claim 1 or 3 or the method according to claim 2 or 3, wherein the biological tissues that are to be decellularized are one or more of biological tissues selected from the group consisting of the heart, the liver, the kidney, the lung, the brain and the spiral cord.

5. The hybrid gel according to any one of claims 1, 3 and 4 or the method according to any one of claims 2 to 4, wherein thrombin is at a concentration of 0.8 U/mL to 12.5 U/mL.

6. The hybrid gel according to any one of claims 1 and 3 to 5, wherein the decellularized biological tissues are the ones prepared by treatment of biological tissues with a high hydrostatic pressure.

7. The method according to any one of claims 2 to 5, wherein the decellularization is conducted by treatment of biological tissues with a high hydrostatic pressure.

8. The method according to any one of claims 2 to 5 and 7, wherein the step giving decellularized biological tissues comprises a step of decellularizing animal-derived biological tissues and a step of microwave irradiation and washing.

9. The method according to any one of claims 2 to 5, 7 and 8, wherein the step of pulverizing the decellularized biological tissues comprises a step of shredding the decellularized biological tissues, a step of lyophilizing or freezing the decellularized biological tissues as shredded, and a step of pulverizing the decellularized biological tissues as lyophilized or frozen.

10. The method according to claim 9, wherein the method further comprises a step of sieving the decellularized biological tissues as pulverized based on a particle size.

11. A medicament comprising the hybrid gel as set forth in any one of claims 1 and 3 to 6 for use in treating diseases.

12. The medicament according to claim 11, wherein the medicament is for use in treating myocardial infarction, diseases caused by nerve damage or skin frostbite.

13. A cell transplantation adjuvant or a cell culture material comprising the hybrid gel as set forth in any one of claims 1 and 3 to 6.

14. The hybrid gel according to any one of claims 1 and 3 to 6 for use in tissue regeneration of an animal tissue.

15. The hybrid gel according to any one of claims 1 and 3 to 5,
the method according to any one of claims 2 to 5 and 7 to 10, or
the hybrid gel for use of claim 14,
wherein the animal is an animal other than human.

16. A method for cell culture comprising culturing cells together with the hybrid gel as set forth in claims 1 and 3 to 6.

17. The cell transplantation adjuvant or cell culture material according to claim 13, or the method for cell culture according to claim 16, wherein the cells are the heart-derived cells, neural progenitor cells, or tissue stem cells.

## Patentansprüche

1. Hybridgel, umfassend ein partikelförmiges dezellularisiertes Gewebe, das durch Pulverisierung von von einem Tier stammenden biologischen Geweben, die dezellularisiert sind (dezellularisierte biologische Gewebe), erhalten wurde, Fibrinogen und Thrombin, wobei Thrombin in einer Konzentration von 0,8 U/ml bis 125 U/ml vorliegt und Fibrinogen in einer Konzentration von 30 mg/ml bis 40 mg/ml vorliegt.

2. Verfahren zur Herstellung eines Hybridgels, umfassend einen Schritt der Dezellularisierung von von einem Tier stammenden biologischen Geweben, um dezellularisierte biologische Gewebe zu ergeben, einen Schritt der Pulverisierung der dezellularisierten biologischen Gewebe, um partikelförmiges dezellularisiertes Gewebe zu ergeben und einen Schritt der Mischung des partikelförmigen dezellularisierten Gewebes mit Fibrinogen und Thrombin, wobei Thrombin in einer Konzentration von 0,8 U/ml bis 125 U/ml vorliegt und Fibrinogen in einer Konzentration von 30 mg/ml bis 40 mg/ml vorliegt.

3. Hybridgel nach Anspruch 1 oder Verfahren nach Anspruch 2, wobei die biologischen Gewebe, die dezellularisiert werden sollen, solche sind, die eine Matrixstruktur haben.

4. Hybridgel nach Anspruch 1 oder 3 oder Verfahren nach Anspruch 2 oder 3, wobei die biologischen Gewebe, die dezellularisiert werden sollen, ein oder mehrere Gewebe ausgewählt aus der Gruppe, bestehend aus dem Herz, der Leber, der Niere, der Lunge, dem Gehirn und dem Rückenmark sind.

5. Hybridgel nach einem der Ansprüche 1, 3 und 4 oder Verfahren nach einem der Ansprüche 2 bis 4, wobei Thrombin in einer Konzentration von 0,8 U/ml bis 12,5 U/ml vorliegt.

6. Hybridgel nach einem der Ansprüche 1 und 3 bis 5, wobei die dezellularisierten biologischen Gewebe, solche sind, die durch die Behandlung von biologischen Geweben mit einem hohen hydrostatischen Druck hergestellt wurden.

7. Verfahren nach einem der Ansprüche 2 bis 5, wobei die Dezellularisierung durch die Behandlung von biologischen Geweben mit einem hohen hydrostatischen Druck vorgenommen wird.

8. Verfahren nach einem der Ansprüche 2 bis 5 und 7, wobei der Schritt, der die dezellularisierten biologischen Gewebe ergibt, einen Schritt der Dezellularisierung von von einem Tier stammenden biologischen Geweben und einen Schritt der Mikrowellenbestrahlung und Waschung umfasst.

9. Verfahren nach einem der Ansprüche 2 bis 5, 7 und 8, wobei der Schritt der Pulverisierung der dezellularisierten biologischen Gewebe einen Schritt der Zerkleinerung der dezellularisierten biologischen Gewebe, einen Schritt der Gefriertrocknung oder des Einfrierens der zerkleinerten dezellularisierten biologischen Gewebe und einen Schritt der Pulverisierung der gefriergetrockneten oder gefrorenen dezellularisierten biologischen Gewebe umfasst.

10. Verfahren nach Anspruch 9, wobei das Verfahren weiterhin einen Schritt des Siebens der pulverisierten dezellularisierten biologischen Gewebe basierend auf einer Partikelgröße umfasst,

11. Medikament, umfassend das Hybridgel nach einem der Ansprüche 1 und 3 bis 6 zur Verwendung in der Behandlung von Krankheiten.

12. Medikament nach Anspruch 11, wobei das Medikament zur Verwendung in der Behandlung eines Herzmuskelinfarkts, Krankheiten, die durch Nervenschäden, hervorgerufen werden oder Hauterfrierungen ist.

13. Zelltransplantationsadjuvans oder Zellkulturmaterial, umfassend das Hybridgel nach einem der Ansprüche 1 und 3 bis 6.

14. Hybridgel nach einem der Ansprüche 1 und 3 bis 6 zur Verwendung in der Geweberegeneration des Gewebes eines Tieres.

15. Hybridgel nach einem der Ansprüche 1 und 3 bis 5,
Verfahren nach einem der Ansprüche 2 bis 5, und 7 bis 10, oder
Hybridgel zur Verwendung nach Anspruch 14,
wobei das Tier ein anderes Tier als der Mensch ist.

16. Verfahren zur Zellkultur, umfassend die Züchtung von Zellen zusammen mit dem Hybridgel nach einem der Ansprüche 1 und 3 bis 6.

17. Zelltransplantationsadjuvans oder Zellkulturmaterial nach Anspruch 13 oder Verfahren zur Zellkultur nach Anspruch 16, wobei die Zellen vom Herzen stammende Zellen, neuronale Vorläuferzellen oder Gewebestammzellen sind.

## Revendications

1. Gel hybride comprenant un tissu décellularisé particulaire obtenu par pulvérisation de tissus biologiques d'origine animale qui sont décellularisés (tissus biologiques décellularisés), du fibrinogène et de la thrombine, dans lequel la thrombine est à une concentration de 0,8 U/ml à 125 U/ml et le fibrinogène est à une concentration de 30 mg/ml à 40 mg/ml.

2. Méthode pour préparer un gel hybride, comprenant une étape de décellularisation de tissus biologiques d'origine animale pour donner des tissus biologiques décellularisés, une étape de pulvérisation desdits tissus biologiques décellularisés pour donner un tissu décellularisé particulaire, et une étape de mélange dudit tissu décellularisé particulaire, de fibrinogène et de thrombine, dans laquelle la thrombine est à une concentration de 0,8 U/ml à 125 U/ml et le fibrinogène est à une concentration de 30 mg/ml à 40 mg/ml.

3. Gel hybride selon la revendication 1 ou méthode selon la revendication 2, dans lequel les tissus biologiques qui doivent être décellularisés sont ceux ayant une structure de matrice.

4. Gel hybride selon la revendication 1 ou 3 ou méthode selon la revendication 2 ou 3, dans lequel les tissus biologiques qui doivent être décellularisés sont un ou plusieurs tissus biologiques choisis dans le groupe constitué par le coeur, le foie, les reins, les poumons, le cerveau et la moelle épinière.

5. Gel hybride selon l'une quelconque des revendications 1, 3 et 4 ou méthode selon l'une quelconque des revendications 2 à 4, dans lequel la thrombine est à une concentration de 0,8 U/ml à 12,5 U/ml.

6. Gel hybride selon l'une quelconque des revendications 1 et 3 à 5, dans lequel les tissus biologiques décellularisés sont ceux préparés par traitement de tissus biologiques avec une forte pression hydrostatique.

7. Méthode selon l'une quelconque des revendications 2 à 5, dans laquelle la décellularisation est effectuée par traitement de tissus biologiques avec une forte pression hydrostatique.

8. Méthode selon l'une quelconque des revendications 2 à 5 et 7, dans laquelle l'étape donnant des tissus biologiques décellularisés comprend une étape de décellularisation de tissus biologiques d'origine animale et une étape d'irradiation aux micro-ondes et de lavage.

9. Méthode selon l'une quelconque des revendications 2 à 5, 7 et 8, dans laquelle l'étape de pulvérisation des tissus biologiques décellularisés comprend une étape de hachage des tissus biologiques décellularisés, une étape de lyophilisation ou de congélation des tissus biologiques décellularisés tels que hachés, et une étape de pulvérisation des tissus biologiques décellularisés tels que lyophilisés ou congelés.

10. Méthode selon la revendication 9, laquelle méthode comprend en outre une étape de tamisage des tissus biologiques décellularisés tels que pulvérisés sur la base de la taille des particules.

11. Médicament comprenant le gel hybride tel que défini dans l'une quelconque des revendications 1 et 3 à 6 pour utilisation dans le traitement de maladies.

12. Médicament selon la revendication 11, ledit médicament étant pour utilisation dans le traitement d'un infarctus du myocarde, de maladies provoquées par un endommagement des nerfs, ou d'une engelure.

13. Adjuvant de greffe cellulaire ou matériau de culture cellulaire comprenant le gel hybride tel que défini dans l'une quelconque des revendications 1 et 3 à 6.

14. Gel hybride selon l'une quelconque des revendications 1 et 3 à 6 pour utilisation dans la régénération tissulaire d'un tissu animal.

15. Gel hybride selon l'une quelconque des revendications 1 et 3 à 5, méthode selon l'une quelconque des revendications 2 à 5 et 7 à 10, ou gel hybride pour utilisation selon la revendication 14,
dans lequel l'animal est un animal autre qu'un humain.

16. Méthode de culture cellulaire comprenant la culture de cellules conjointement avec le gel hybride tel que défini dans les revendications 1 et 3 à 6.

17. Adjuvant de greffe cellulaire ou matériau de culture cellulaire selon la revendication 13, ou méthode de culture cellulaire selon la revendication 16, dans lequel les cellules sont des cellules d'origine cardiaque, des cellules progénitrices neurales, ou des cellules souches tissulaires.
